(19) Europäisches Patentamt / European Patent Office / Office européen des brevets

(11) **EP 4 343 414 B1**

(12) **EUROPÄISCHE PATENTSCHRIFT**

(45) Veröffentlichungstag und Bekanntmachung des Hinweises auf die Patenterteilung:
**25.06.2025   Patentblatt 2025/26**

(21) Anmeldenummer: **22197622.8**

(22) Anmeldetag: **26.09.2022**

(51) Internationale Patentklassifikation (IPC):
**G02C 7/04** *(2006.01)*      **A61F 2/16** *(2006.01)*

(52) Gemeinsame Patentklassifikation (CPC):
**G02C 7/044; G02C 7/042;** A61F 2/1618;
G02C 2202/20; G02C 2202/22

(54) **MULTIFOKALE LINSE**

MULTIFOCAL LENS

LENTILLE MULTIFOCALE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**27.03.2024   Patentblatt 2024/13**

(73) Patentinhaber: **Werner  Fiala**
**1180 Wien (AT)**

(72) Erfinder:
  • **FIALA, Werner**
  **1180 Wien (AT)**

  • **PINGITZER, Johann**
  **7022 Schattendorf (AT)**

(74) Vertreter: **Weiser Voith Gugler
Patentanwälte Partnerschaft
Kopfgasse 7
1130 Wien (AT)**

(56) Entgegenhaltungen:
**DE-A1- 102020 215 362      US-A- 5 760 871
US-B1- 6 536 899**

**Beschreibung**

Die vorliegende Erfindung betrifft eine multifokale Linse mit zumindest drei Hauptbrechkräften, welche mehrere konzentrische, jeweils aneinander angrenzende ringförmige Hauptzonen aufweist, von denen jede in eine innere und eine äußere ringförmige Subzone verschiedener refraktiver Brechkraft unterteilt ist, wobei die Linse frei von geometrischen Stufen zwischen allen Subzonen ist.

Derartige Linsen finden häufig als ophthalmische Linse Verwendung, z.B. als Kontaktlinse, Intraokularlinse (IOL), Intrakorneallinse oder Brillenlinse.

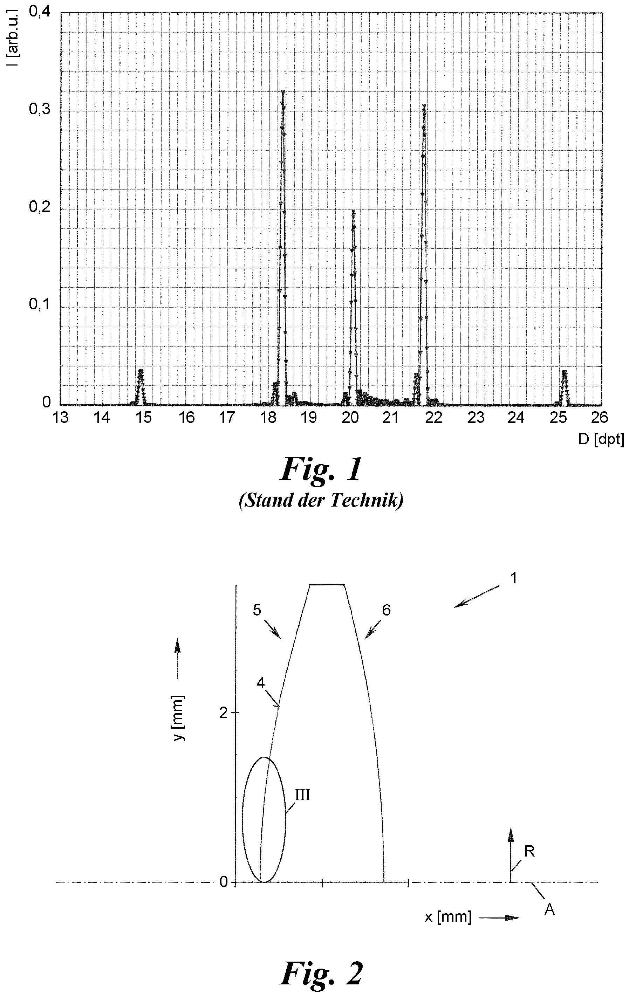

Trifokale Linsen sind seit langer Zeit bekannt. In der Mehrzahl der Fälle handelt es sich um diffraktive Linsen mit ringförmigen Zonen gleicher Fläche, sog. "Fresnel'schen Zonen", bei denen zwischen den Zonen geometrische Stufen vorgesehen sind. Bei derartigen trifokalen Linsen ist die Höhe der Stufen in der Regel abwechselnd verschieden groß. Fig. 1 zeigt die Brechkraftverteilung ("through focus response", TFR) einer solchen Linse mit 28 Zonen, bei der die Stufen zwischen den flächengleichen ringförmigen Zonen abwechselnd hoch sind, sodass die optischen Weglängendifferenzen $0,65 \cdot \lambda$ und $1,35 \cdot \lambda$ betragen, wobei $\lambda$ eine Lichtwellenlänge bezeichnet. Ferner sind Linsen bekannt, die in Radialrichtung Wellenform (DE 10 2020 215 362 A1) und/oder Rechteckform (US 5760871) aufweisen, welche im radialen Verlauf Asymmetrien, Abflachungen bzw. Änderungen der Periodizität und dabei im Wesentlichen ein gleichbleibendes Höhenprofil haben. Derartige Stufen, Wellen oder Rechtecke sind jedoch aufwändig herzustellen, was hohe Fertigungskosten nach sich zieht.

Weiters sind trifokale refraktiv-diffraktive Linsen bekannt, bei denen die Stufen durch sog. "Phasensubzonen" kleiner Fläche ersetzt werden, deren refraktive Brechkräfte sich wesentlich von den refraktiven Brechkräften der anderen, sog. "Phasenhauptzonen" unterscheiden und dadurch einen entsprechenden optischen Weglängenunterschied zwischen den Phasenhauptzonen bewerkstelligen (z.B. EP 1 194 797 B1, EP 2 564 265 B1). Derartige trifokale Linsen weisen von innen nach außen einen komplexen Brechkräfte-Verlauf und einen variierenden Verlauf der Flächenverhältnisse zwischen den Phasensubzonen und den Phasenhauptzonen auf und sind daher in der Regel aufwändig zu fertigen.

Die Erfindung setzt sich zum Ziel, eine multifokale Linse mit zumindest drei Hauptbrechkräften zu schaffen, die einfach und kostengünstig zu fertigen ist.

Dieses Ziel wird mit einer multifokalen Linse der einleitend genannten Art erreicht, die sich gemäß der Erfindung dadurch auszeichnet, dass die refraktiven Brechkräfte aller inneren Subzonen untereinander jeweils gleich sind und die refraktiven Brechkräfte aller äußeren Subzonen untereinander jeweils gleich sind, und dass alle inneren und äußeren Subzonen ihre jeweilige Hauptzone in einem gleichen Flächenverhältnis teilen, welches im Bereich von 30:70 bis 70:30 liegt.

Grundlage der Erfindung ist die explizite Berücksichtigung der Interferenzphänomene zwischen Licht aus den verschiedenen Subzonen bzw. Hauptzonen. Die Erfindung schafft durch die Kombination der alternierenden Subzonen-Brechkräfte und der speziellen Flächenverhältnisse zwischen inneren und äußeren Subzonen eine multifokale Linse, welche gleich drei oder mehr Hauptbrechkräfte aufweisen kann, von denen zumindest eine diffraktiv ist. Aufgrund des einfachen Oberflächenprofil-Verlaufs mit alternierenden refraktiven Brechkräften der Subzonen, mit gleichen Flächenverhältnissen der die Hauptzonen bildenden Subzonenpaare und ohne geometrische Stufen, ist die Multifokallinse der Erfindung besonders einfach und kostengünstig zu fertigen. Darüberhinaus ermöglicht die erfindungsgemäße Linse eine Vielzahl an Aufteilungen der Lichtintensität auf ihre Hauptbrechkräfte, z.B. eine gleichmäßige Aufteilung für eine gute Sicht in der Ferne, der Mitteldistanz und der Nähe.

In einer bevorzugten Ausführungsform haben alle Hauptzonen jeweils die gleiche Fläche. Dadurch weisen die Hauptzonen die Flächenverhältnisse einer Fresnel-Zonenlinse auf, was die Erzeugung der zumindest einen diffraktiven Hauptbrechkraft durch Interferenzen zwischen den Sub- und Hauptzonen vereinfacht. In einer dazu alternativen Ausführungsform nehmen die Flächen der Hauptzonen von innen nach außen monoton zu oder ab, wodurch den einzelnen Hauptbrechkräften eine Abhängigkeit von einer Blendengröße, z.B. einer Lochblende oder der Augenpupille, verliehen werden kann.

Die diffraktiven Hauptbrechkräfte der Linse können insbesondere für sichtbares Licht besonders einfach erzeugt werden, wenn die Fläche jeder Hauptzone kleiner als $2,2\pi$ mm$^2$ ist, bevorzugt kleiner als $\pi$ mm$^2$, besonders bevorzugt kleiner als $2\pi/3$ mm$^2$.

In einer fertigungstechnisch besonders günstigen Ausführungsform liegt das genannte Flächenverhältnis bevorzugt im Bereich von 40:60 bis 60:40 und ist besonders bevorzugt 50:50.

Die Linse kann eine beliebige Mehrzahl an Hauptzonen aufweisen, z.B. mehr als 50 oder mehr als 100. Wenn die Linse 5 bis 50 Hauptzonen aufweist, bevorzugt 10 bis 20 Hauptzonen, kann ihr Oberflächen-Profil einfach gehalten werden, während gleichzeitig ihre zumindest eine diffraktive Hauptbrechkraft durch Lichtbeugung an den Sub- und Hauptzonen mit scharfem Fokus ausgebildet sein kann.

In einer weiteren bevorzugten Ausführungsform weist die Linse als Hauptbrechkräfte eine Fern-, eine Mittel- und eine Nahbrechkraft auf, wobei die Differenz zwischen der Nahbrechkraft und der Fernbrechkraft im Bereich von 1 bis 6 Dioptrien liegt, bevorzugt im Bereich von 2,0 bis 4,5 Dioptrien. Auf diese Weise können bei Verwendung der Linse,

**EP 4 343 414 B1**

insbesondere als IOL, nahe und ferne Objekte gut fokussiert werden. Wenn dabei zusätzlich die Differenz zwischen der Mittelbrechkraft und der Fernbrechkraft im Bereich von 1,4 bis 2 Dioptrien liegt, bevorzugt im Bereich von 1,6 bis 1,8 Dioptrien, können auch intermediäre Objekte gut fokussiert werden.

Grundsätzlich könnte die Linse eine oder zwei refraktive Hauptbrechkräfte aufweisen, welche mit der refraktiven Brechkraft der inneren und/oder äußeren Subzonen zusammenfällt/zusammenfallen. In einer vorteilhaften Ausführungsform bilden alle Subzonen in Kombination ein Beugungsgitter, das die zumindest drei Hauptbrechkräfte der Linse durch Diffraktion erzeugt. Dadurch fällt keine der Hauptbrechkräfte der Linse mit einer der refraktiven Brechkräfte ihrer Subzonen zusammen und die Hauptbrechkräfte können rein durch Berücksichtigung der Interferenzphänomene zwischen Licht aus verschiedenen Subzonen bzw. Hauptzonen festgelegt werden.

In einer günstigen Kombination der beiden letztgenannten Ausführungsformen kann die Fernbrechkraft durch die negative erste Diffraktionsordnung (auch "Beugungsordnung" genannt) der Linse erzeugt sein. Während von der Verwendung dieser Diffraktionsordnung für die Fernbrechkraft herkömmlicherweise aufgrund ihrer chromatischen longitudinalen Aberration abgeraten wird, wurde nun erstmals erkannt, dass ebendiese chromatische longitudinale Aberration in vorteilhafter Weise dazu verwendet werden kann, die in der menschlichen oder tierischen Augenlinse vorhandene refraktive chromatische Aberration nachzubilden bzw. zu kompensieren. Insbesondere kann hierfür die negative erste Diffraktionsordnung der Linse für Licht zwischen 450 nm und 650 nm eine diffraktive longitudinale chromatische Aberration aufweisen, welche im Bereich von 0,1 bis 1,2 Dioptrien liegt, bevorzugt im Bereich von 0,3 bis 0,7 Dioptrien, besonders bevorzugt im Bereich von 0,35 bis 0,55 Dioptrien.

In einer vorteilhaften Ausführungsform der Linse, insbesondere als IOL, liegt die Brechkraft der inneren Subzonen oder die Brechkraft der äußeren Subzonen im Bereich von -2,5 bis 2,5 Dioptrien um die kleinste der Hauptbrechkräfte, bevorzugt im Bereich von -2,0 bis 2,0 Dioptrien um die kleinste der Hauptbrechkräfte, wodurch die Hauptbrechkräfte der Linse im Bereich der Brechkraft der menschlichen Augenlinse liegen können. Zu demselben Zweck kann in einer optional damit kombinierbaren, weiteren vorteilhaften Ausführungsform die Brechkraft der inneren Subzonen oder die Brechkraft der äußeren Subzonen im Bereich von -2,5 bis 2,5 Dioptrien um die größte der Hauptbrechkräfte liegen, bevorzugt im Bereich von -2,0 bis 2,0 Dioptrien um die größte der Hauptbrechkräfte.

Die Subzonen können allesamt homogen sein. Alternativ dazu kann zumindest eine innere oder äußere Subzone in Teilzonen unterteilt sein, deren gemittelte refraktive Brechkräfte der refraktiven Brechkraft der jeweiligen Subzone entsprechen. Auf diese Weise können eine oder gleich mehrere Subzonen z.B. eine diskrete oder stetige Brechkraftverteilung aufweisen, was eine Vielzahl an Fertigungsmöglichkeiten der Linse und eine Vielzahl an erhältlichen Beugungsmustern ermöglicht.

Die Erfindung wird nachstehend anhand von in den beigeschlossenen Zeichnungen dargestellten Ausführungsbeispielen näher erläutert. In den Zeichnungen zeigen:

Fig. 1 eine Brechkraftverteilung ("through focus response", TFR), wie sie mit einer Trifokallinse gemäß dem Stand der Technik bei einer Licht-Wellenlänge von 550 nm erhalten wird, in einem Intensitäts-Brechkraft-Diagramm;

Fig. 2 eine erste Ausführungsform einer erfindungsgemäßen Trifokallinse in einem Halbschnitt normal zu ihrer Achse A der Rotationssymmetrie;

Fig. 3 einen zentralen Ausschnitt III der Linse von Fig. 2 in einem Halbschnitt normal zur Achse A mit zwecks besserer Erkennbarkeit gespreizter x-Achse;

Fig. 4 eine Brechkraftverteilung, wie sie mit der Linse der Fig. 2 und 3 bei einer Licht-Wellenlänge von 550 nm erhalten wird, in einem Intensitäts-Brechkraft-Diagramm;

Fig. 5 eine Kontrastübertragungsfunktion ("modulation transfer function", MTF), wie sie mit der Linse der Fig. 2 und 3 erhalten wird, in einem Kontrast-Linien-Diagramm;

Fig. 6 eine Brechkraftverteilung, wie sie mit einer beispielhaften Variante der Linse der Fig. 2 und 3 bei einer Licht-Wellenlänge von 550 nm erhalten wird, in einem Intensitäts-Brechkraft-Diagramm;

Fig. 7 eine Brechkraftverteilung, wie sie mit einer erfindungsgemäßen Trifokallinse gemäß einer zweiten Ausführungsform bei einer Licht-Wellenlänge von 550 nm erhalten wird, in einem Intensitäts-Brechkraft-Diagramm;

Fig. 8 eine Brechkraftverteilung, wie sie mit einer erfindungsgemäßen Trifokallinse gemäß einer dritten Ausführungsform bei einer Licht-Wellenlänge von 550 nm erhalten wird, in einem Intensitäts-Brechkraft-Diagramm;

Fig. 9 eine Brechkraftverteilung, wie sie mit der Linse der Fig. 2 und 3 bei polychromatischem Licht mit Wellenlängen von 450 nm bis 650 nm erhalten wird, in einem Intensitäts-Brechkraft-Diagramm;

die Fig. 10a und 10b drei Brechkraftverteilungen, wie sie mit erfindungsgemäßen Trifokallinsen, welche sich in den Flächenverhältnissen ihrer aufeinanderfolgender Hauptzonen unterscheiden, bei einer Licht-Wellenlänge von 550 nm (Fig. 10a) bzw. bei polychromatischem Licht mit Wellenlängen von 450 nm bis 650 nm (Fig. 10b) erhalten werden, jeweils in einem Intensitäts-Brechkraft-Diagramm;

Fig. 11 die Abhängigkeit der Brechkraft des menschlichen Auges von der Licht-Wellenlänge in einem Brechkraft-Wellenlängen-Diagramm; und

Fig. 12 die diffraktive longitudinale chromatische Aberration anhand zweier Brechkraftverteilungen, wie sie mit der

3

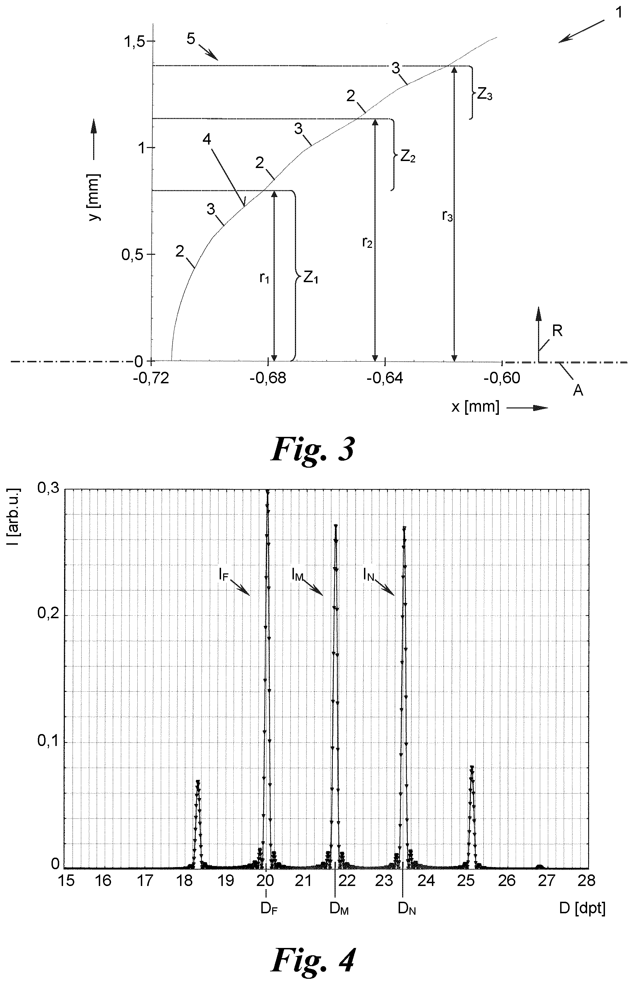

Linse der Fig. 2 und 3 bei Licht-Wellenlängen von 450 nm und 650 nm erhalten werden, in einem Intensitäts-Brechkraft-Diagramm.

Zu Fig. 1, welche den Stand der Technik zeigt, wird auf die einleitenden Ausführungen verwiesen.

Die Fig. 2 und 3 zeigen eine erfindungsgemäße multifokale Linse 1 mit zumindest drei Hauptbrechkräften, insbesondere einer Fernbrechkraft $D_F$, einer Mittelbrechkraft $D_M$ und einer Nahbrechkraft $D_N$ (Fig. 4) für die Sicht auf ferne, intermediäre bzw. nahe Objekte. Die Linse 1 kann z.B. als ophthalmische Linse verwendet werden, z.B. als Kontaktlinse, Intraokularlinse (IOL), Intrakorneallinse oder Brillenlinse, oder als optisches Element, z.B. als Spiegel, Sammel- oder Zerstreuungslinse. Die Linse 1 kann aus jedem hiefür geeigneten Material sein, z.B. aus Glas, Acryl, Silikon, Hydrogel, Polymethylmethacrylat (PMMA), usw.

Die Linse 1 weist mehrere (zwei, drei oder mehr) um ihre Achse A der Rotationssymmetrie konzentrische Hauptzonen $Z_i$ (i = 1, 2, ..., I) auf, welche jeweils aneinander angrenzen. D.h. von innen nach außen (in Fig. 3: in Radialrichtung R) grenzt die zweite Hauptzone $Z_2$ mit ihrem Innenradius $r_1$ (gemessen von der optischen Achse A) an die erste Hauptzone $Z_1$ an, die dritte Hauptzone $Z_3$ mit ihrem Innenradius $r_2$ an die zweite Hauptzone $Z_2$, usw. - stets ohne Zwischenschaltung weiterer optischer Bereiche. Die Anzahl I > 1 der Hauptzonen $Z_i$ kann abhängig vom Gesamtdurchmesser der Linse 1 und der gewünschten Differenz zwischen Nahbrechkraft $D_N$ und Fernbrechkraft $D_F$ bestimmt werden und liegt zumeist im Bereich von 5 bis 50, insbesondere im Bereich von 10 bis 20.

Jede Hauptzone $Z_i$ ist in eine innere Subzone 2 und eine äußere Subzone 3 unterteilt, welche voneinander verschiedene refraktive Brechkräfte aufweisen, hierin mit den Formelzeichen "$D_1$" bzw. "$D_2$" bezeichnet. Dabei sind die refraktiven Brechkräfte $D_1$ aller inneren Subzonen 2 bzw. die refraktiven Brechkräfte $D_2$ aller äußeren Subzonen 3 untereinander jeweils gleich, also hat jede innere Subzonen 2 die refraktive Brechkraft $D_1$ und jede äußere Subzone 3 die refraktive Brechkraft $D_2$.

Ferner ist in jeder der Hauptzonen $Z_i$ das Flächenverhältnis zwischen ihrer inneren und ihrer äußeren Subzone 2, 3 gleich und im Bereich von 30:70 bis 70:30. Wird der flächenmäßige Anteil jeder inneren Subzone 2 an ihrer Hauptzone $Z_i$ mit $p_1$ bezeichnet und der flächenmäßige Anteil jeder äußeren Subzone 3 an ihrer Hauptzone $Z_i$ mit $p_2$, gilt daher die Ungleichung 30:70 $\leq p_1:p_2 \leq$ 70:30. In den meisten Ausführungsformen liegt das Flächenverhältnis $p_1:p_2$ zwischen 40:60 und 60:40, in manchen Ausführungsformen ist es im Wesentlichen 50:50.

Wie in Fig. 3 ersichtlich, weist die Linse 1 keine Stufen zwischen allen Subzonen 2, 3 auf, also weder zwischen den Hauptzonen $Z_i$ noch zwischen deren jeweiligen Subzonen 2, 3. Jene Linsenoberfläche 4, welche die Multifokalität der Linse 1 bewirkt, ist also stetig.

Zumindest eine der Hauptbrechkräfte $D_F$, $D_M$, $D_N$ der Linse 1 ist diffraktiv, also durch Beugungseffekte an der Linse 1 erzeugt. Grundlage der hier offenbarten Linse 1 ist somit die explizite Berücksichtigung der Interferenzphänomene zwischen Licht aus den verschiedenen Subzonen 2, 3 bzw. Hauptzonen $Z_i$. Hiefür kann die Fläche jeder Hauptzone $Z_i$ z.B. kleiner als $2,2\pi$ mm$^2$ gewählt werden, z.B. kleiner als $\pi$ mm$^2$ oder kleiner als $2\pi/3$ mm$^2$. In manchen Ausführungsformen bilden alle Subzonen 2, 3 in Kombination ein Beugungsgitter, das gleich alle (hier: drei; alternativ: vier oder mehr) Hauptbrechkräfte $D_F$, $D_M$, $D_N$, ... der Linse 1 erzeugt. Dann sind alle Hauptbrechkräfte $D_F$, $D_M$, $D_N$, ... der Linse 1 diffraktiv und die refraktiven Brechkräfte $D_1$, $D_2$ in den Subzonen 2, 3 stimmen in den meisten Ausführungsformen mit keiner der resultierenden Hauptbrechkräfte $D_F$, $D_M$, $D_N$, ... der multifokalen Linse 1 überein.

In der in den Fig. 2 und 3 dargestellten beispielhaften Ausführungsform der Linse 1 ist diese eine Intraokularlinse (IOL) mit einem Brechungsindex von 1,458. Die Hauptbrechkräfte $D_F$, $D_M$, $D_N$ der Linse 1 sind 20, 21,7 und 23,4 Dioptrien, die inneren Subzonen 2 haben eine refraktive Brechkraft $D_1$ von 19,8 Dioptrien und die äußeren Subzonen 3 eine Brechkraft $D_2$ von 23,8 Dioptrien. Auf der Vorderseite 5 der Linse 1 befindet sich die die Multifokalität erzeugende Linsenoberfläche 4 mit allen Subzonen 2, 3. Alternativ oder zusätzlich kann auch die Rückseite 6 der Linse 1 eine solche Linsenoberfläche 4 mit Haupt- und Subzonen $Z_i$, 2, 3 aufweisen. In einer Ausführungsform ist die Linse 1 eine torische Linse, wobei die der Linsenoberfläche 4 abgewandte Linsenoberfläche die Form eines Torus-Käppchens hat.

In der maßstäblichen Darstellung der Fig. 2 sind die in den einzelnen Subzonen 2, 3 vorhandenen verschiedenen refraktiven Brechkräfte $D_1$, $D_2$ nicht zu erkennen. Deshalb ist in Fig. 3 ein zentraler Ausschnitt III der Linsenoberfläche 4 so dargestellt, dass die x-Achse parallel zur optischen Achse A um den Faktor 13,3 gestreckt ist, um die verschiedenen Krümmungen in den einzelnen Hauptzonen $Z_i$ deutlich zu machen.

Aus den Darstellungen der Fig. 2 und 3 ist erkennbar, dass die hier offenbarte Linse 1 einfacher herzustellen ist als z.B. eine Diffraktionslinse nach dem Stand der Technik mit Stufen zwischen den einzelnen Zonen. Insbesondere kann die Linse 1 auf diese Weise stetige Linsenoberflächen 4 auf der Vorder- und/oder der Rückseite 5, 6 haben.

In der beispielhaften Ausführungsform der Fig. 2 und 3 hat die Linse 1 vierzehn Hauptzonen $Z_i$ gleicher Fläche (d.h. Fresnel'sche Zonen) auf einem Durchmesser von 6,02 mm. Die zentrale Hauptzone $Z_1$ besitzt einen Durchmesser $d_1$ = $2r_1$ von 1,6088 mm; die an diese Hauptzone $Z_1$ anschließende ringförmige zweite Hauptzone $Z_2$ besitzt einen Innendurchmesser $d_1$ = $2r_1$ von 1,6088 mm und einen Außendurchmesser $d_2$ = $2r_2$ von $1{,}6088\sqrt{2}$ mm; die dritte

Hauptzone $Z_3$ besitzt einen Außendurchmesser $d_3 = 2r_3$ von $1,6088\sqrt{3}$ mm; und die i-te Hauptzone $Z_i$ besitzt einen Innendurchmesser $d_{i-1} = 2r_{i-1}$ von $1,6088\sqrt{i-1}$ mm und einen Außendurchmesser $d_i = 2r_i$ von $1,6088\sqrt{i}$ mm. Im betrachteten Beispiel betragen die Flächenanteile $p_1$ der inneren Subzonen 2 jeweils 52,5 % und die Flächenanteile $p_2$ der äußeren Subzonen 3 jeweils 47,5 % der Fläche der jeweiligen Hauptzone $Z_i$.

Die resultierende Brechkraftverteilung ("through focus response", TFR) der Linse 1 ist in Fig. 4 in einem Diagramm der Intensität I über der Brechkraft D dargestellt. Wie aus Fig. 4 ersichtlich, betragen die integrierten Intensitäten $I_F$, $I_M$, $I_N$ in den drei Hauptbrechkräften $D_F = 20$ Dioptrien, $D_M = 21{,}7$ Dioptrien und $D_N = 23{,}4$ Dioptrien insgesamt 84 % der gesamten integrierten Intensität, wobei die restlichen 16% Intensität in Nebenmaxima vorhanden sind, die bei 18,3 bzw. 25,1 Dioptrien aufscheinen.

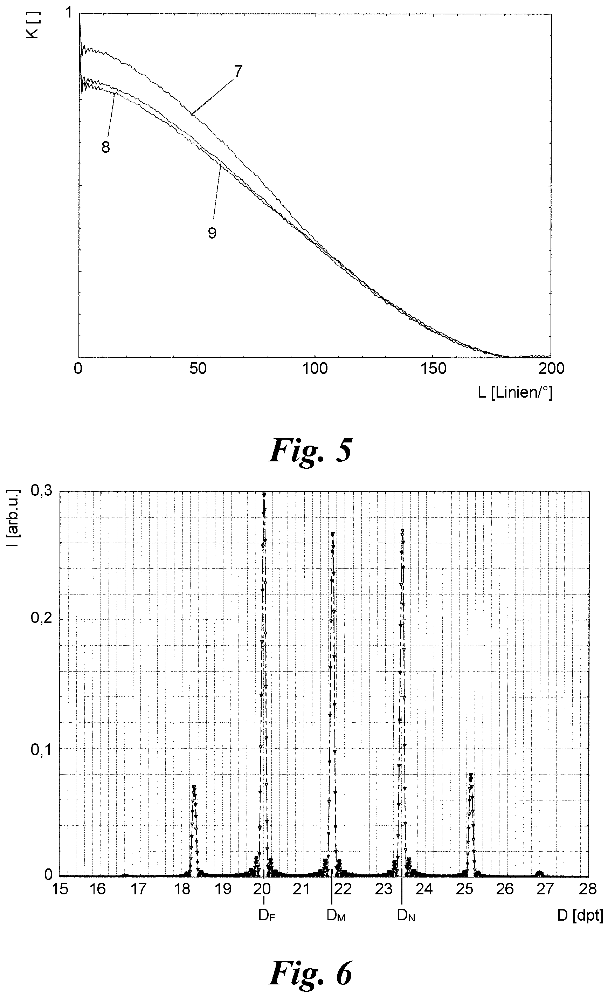

Zur Beurteilung der Abbildungseigenschaften wird oftmals die Kontrastübertragungsfunktion ("modulation transfer function", MTF) herangezogen. In Fig. 5 sind für die Linse 1 der Fig. 2 und 3 die MTFs in den drei Hauptbrechkräften $D_F$, $D_M$, $D_N$ für eine Licht-Wellenlänge von 550 nm als Kurven 7 - 9 des Kontrastes K über der Liniendichte L (Linien pro Grad) dargestellt, und zwar als Kurve 7 für die Fernbrechkraft $D_F$, als Kurve 8 für die Mittelbrechkraft $D_M$ und als Kurve 9 für die Nahbrechkraft $D_N$. Bei der gegenständlichen Linse 1 sind die MTFs für die jeweiligen Hauptbrechkräfte in der Mitteldistanz und der Nahdistanz (Kurven 8 und 9) praktisch gleich; die MTF für die Fernbrechkraft (Kurve 7) ist etwas höher als für die beiden anderen Hauptbrechkräfte.

Zu beachten ist, dass eine herkömmliche diffraktive Trifokallinse mit gleichem Brechkraftabstand von 3,4 Dioptrien zwischen Nahbrechkraft $D_N$ und Fernbrechkraft $D_F$ bei gleichem Durchmesser von 6,02 mm 28 Fresnelzonen mit 27 Stufen, d.h. Unstetigkeiten zwischen diesen Zonen, benötigen würde, um die in Fig. 1 dargestellte Brechkraftverteilung zu erhalten. Die Linse 1 der vorliegenden Offenbarung benötigt hingegen keine Stufen zwischen den Haupt-zonen $Z_i$ oder Subzonen 2, 3.

Für die beispielhafte Linse 1 der Fig. 2 und 3 wurden folgende Parameter gewählt: $D_1 = 19{,}8$ Dioptrien, $D_2 = 23{,}8$ Dioptrien, $D_F = 20$ Dioptrien, $D_M = 21{,}7$ Dioptrien, $D_N = 23{,}4$ Dioptrien, $p_1 = 0{,}525$ und $p_2 = 0{,}475$. Wie zu sehen ist, ist jede der resultierenden Hauptbrechkräfte $D_F$, $D_M$, $D_N$ der Linse 1 ungleich den refraktiven Brechkräften $D_1$ und $D_2$ und damit auf Interferenzphänomene zurückzuführen.

Weiters gilt für die Mittel-Hauptbrechkraft $D_M$ die Beziehung:

$$D_M \; = \; D_1 \; \cdot \; p_1 \; + \; D_2 \; \cdot \; p_2 \qquad\qquad\qquad (1)$$

Die Differenz $D_N - D_F$ ist nicht von der Wahl der refraktiven Brechkräfte $D_1$ und $D_2$ abhängig, sondern lediglich von den Flächen der Haupt- bzw. Subzonen 2, 3 und kann beispielsweise bei Hauptzonen 2 gleicher Fläche gemäß $D_N - D_F = (2, 2\pi \text{ mm}) / (F \cdot 10^3)$ bestimmt werden, wobei F die Fläche der Hauptzonen in mm$^2$ bezeichnet.

Eine der refraktiven Brechkräfte $D_1$ oder $D_2$ ist daher in Grenzen frei wählbar. Wird etwa $D_1$ frei gewählt, so gilt:

$$D_1 \; \cdot \; p_1 \; + \; D_2 \; \cdot \; p_2 \; = \; D_M \; = \; D_1 \; \cdot \; p_1 \; + \; D_2 \; \cdot \; (1 \; - \; p_1) \qquad (2)$$

woraus folgt:

$$D_2 \; = \; (D_M \; - \; D_1 \; \cdot \; p_1) \; / \; (1 \; - \; p_1). \qquad\qquad (3)$$

Wird etwa ein Wert von 20,5 Dioptrien für $D_1$ genommen und soll $D_M$ wieder 21,7 Dioptrien betragen, so ergibt für die Flächenanteile $p_1 = 0{,}525$ und $p_2 = 0{,}475$ Gleichung (3) für $D_2$ den Wert 23,0263 Dioptrien.

Anstelle von $D_1$ könnte auch $D_2$ vorgeben werden und es ergibt sich dann $D_1$ aus der Beziehung

$$D_1 \; = \; (D_M \; - \; D_2 \; \cdot \; p_2) \; / \; ( \; 1 \; - \; p_2) \qquad\qquad (4)$$

Mit der Linse 1 wird wie erörtert z.B. Trifokalität durch I Hauptzonen $Z_i$ erzielt, die jeweils in zwei Subzonen 2, 3 unterteilt sind, die unterschiedliche refraktive Brechkräfte $D_1$, $D_2$ aufweisen. Der Vollständigkeit halber sei angeführt, dass die einzelnen Subzonen 2, 3 auch in weitere Teilzonen (nicht gezeigt) unterteilt sein könnten. Wenn die über die Subzonen-Fläche gemittelten Brechkräfte aller Teilzonen einer Subzone 2 bzw. 3 der refraktiven Brechkraft $D_1$ bzw. $D_2$ entsprechen, ist die Brechkraftverteilung im Wesentlichen die gleiche wie wenn nur eine einzige Brechkraft $D_1$ bzw. $D_2$ pro Subzone 2, 3 verwendet wird. Allgemein können die einzelnen Brechkräfte $D_1$, $D_2$ der Subzonen 2, 3 jeweils durch eine beliebige stetige Brechkraftverteilung innerhalb der Subzone 2, 3 ersetzt werden, solange der über die Subzonen-Fläche gebildete Mittelwert dieser Brechkraftverteilung mit der erforderlichen einzelnen Brechkraft $D_1$ bzw. $D_2$ übereinstimmt.

In Fig. 6 ist die Brechkraftverteilung für eine Variante der Linse 1 der Fig. 2 und 3 dargestellt, bei der jede Subzone 2, 3 aus jeweils zwei flächengleichen Teilzonen besteht. Die beiden Teilzonen jeder inneren Subzone 2 haben 19,5 bzw. 20,1 Dioptrien und die beiden Teilzonen jeder äußeren Subzone 3 haben 23,4 bzw. 24,2 Dioptrien. Der Mittelwert jeder Subzone 2 ist somit 19,8 Dioptrien und der Mittelwert jeder Subzone 3 ist 23,8 Dioptrien, die übrigen Parameter entsprechen jenen der Linse 1 der Fig. 2 und 3. Wie zu sehen ist, sind die Brechkraftverteilungen der Fig. 4 und 6 praktisch identisch. Alternativ können die Subzonen 2, 3 jeweils variierende Brechkraftprofile aufweisen, deren jeweilige Mittelwerte durch $D_1$ bzw. $D_2$ gegeben sind.

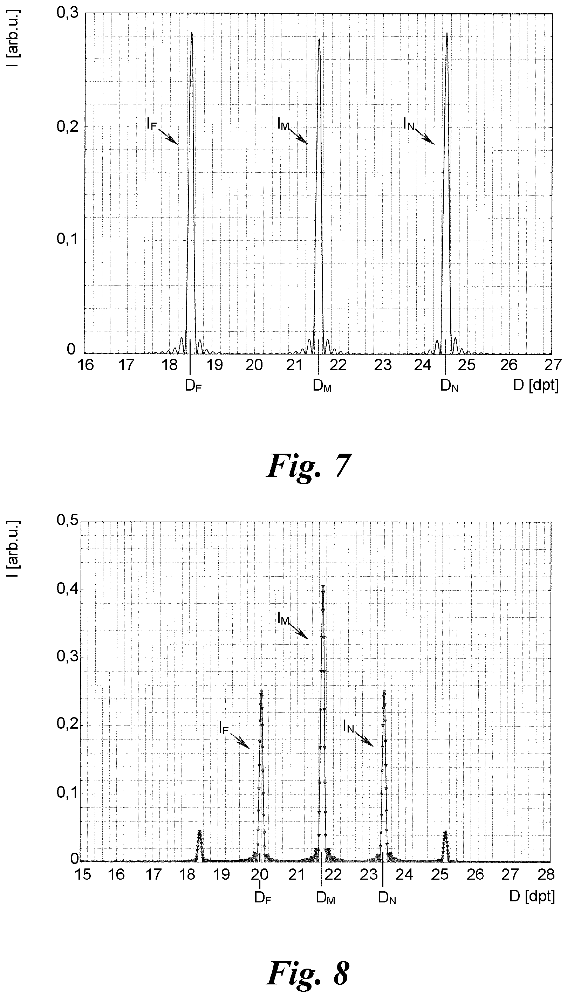

In Fig. 7 ist für monochromatisches Licht von 550 nm die Brechkraftverteilung einer weiteren Ausführungsform der Linse 1 dargestellt, bei der die flächenmäßigen Anteile der Subzonen 2, 3 an jeder Hauptzone $Z_i$ gleich sind, d.h. $p_1 = p_2 = 0,5$. Die inneren Subzonen 2 haben hier eine refraktive Brechkraft $D_1$ von 18 Dioptrien und die äußeren Subzonen 3 eine refraktive Brechkraft $D_2$ von 25 Dioptrien. Wie ersichtlich weist die Linse 1 drei Hauptbrechkräfte $D_F$, $D_M$, $D_N$ von 18,5, 21,5 und 24,5 Dioptrien mit jeweils annähernd gleichen Intensitäten $I_F$, $I_M$, $I_N$ auf.

In den bisher beschriebenen Ausführungsformen stimmen die resultierenden Hauptbrechkräfte $D_F$, $D_M$, $D_N$ der Linse 1 mit den refraktiven Brechkräften $D_1$, $D_2$ der Subzonen nicht überein. In Fig. 8 ist die Brechkraftverteilung einer alternativen Ausführungsform gezeigt, in welcher die Fernbrechkraft $D_F$ mit der refraktiven Brechkraft $D_1$ der inneren Subzonen 2 und die Nahbrechkraft $D_N$ mit der refraktiven Brechkraft $D_2$ der äußeren Subzonen 3 übereinstimmt, wobei beide Arten von Subzonen 2, 3 die gleichen flächenmäßigen Anteile an jeder Hauptzone $Z_i$ aufweisen, also $p_1 = p_2 = 0,5$ gilt. Wie in Fig. 8 zu sehen ist, hat die Mittelbrechkraft $D_M$ eine Intensität $I_M$, die höher als die Intensitäten $I_F$, $I_N$ der beiden anderen Hauptbrechkräfte $D_F$, $D_N$ ist.

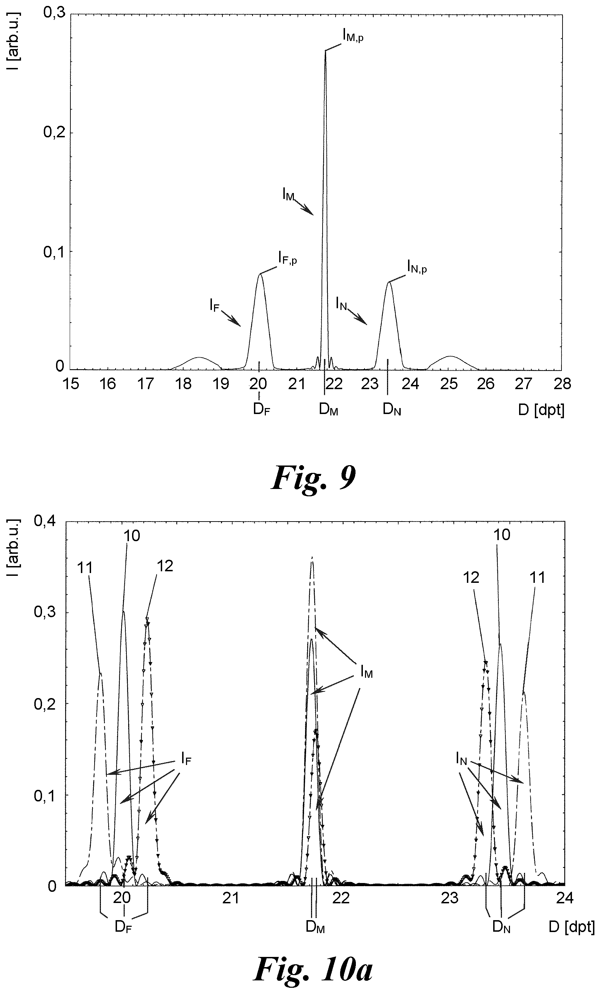

In den bisherigen Ausführungen wurde die Funktionsweise der Linse 1 für monochromatisches Licht erläutert. In Fig. 9 ist die Brechkraftverteilung der Linse 1 der Fig. 2 und 3 für polychromatisches Licht gezeigt, dessen Spektrum sich von 450 bis 650 nm erstreckt. Die Fernbrechkraft $D_F$ von 20 Dioptrien entspricht der negativen ersten Diffraktionsordnung der Linse 1, die Nahbrechkraft $D_N$ von 23,4 Dioptrien der positiven ersten Diffraktionsordnung und die Mittelbrechkraft $D_M$ von 21,7 Dioptrien der nullten Diffraktionsordnung. Die positive und die negative erste Diffraktionsordnung weisen jeweils eine diffraktive chromatische Aberration auf, wodurch die Peak-Intensitäten $I_{F,p}$, $I_{N,p}$ in diesen Diffraktionsordnungen für polychromatisches Licht kleiner sind als die Peak-Intensität $I_{M,p}$ in der nullten Diffraktionsordnung. Wie Fig. 9 zu entnehmen ist, sind die integrierten Intensitäten $I_F$, $I_M$, $I_N$ in den einzelnen Hauptbrechkräften $D_F$, $D_M$, $D_N$ annähernd gleich, wodurch die Abbildungsqualitäten in den drei Hauptbrechkräften $D_F$, $D_M$, $D_N$ vergleichbar sind.

Im gezeigten Beispiel ist die Linse 1 aus einem Material gefertigt, das ihr eine vernachlässigbar kleine refraktive chromatische Aberration verleiht, d.h. dass sein Brechungsindex im Wesentlichen unabhängig von der Wellenlänge des Lichts ist. Beispielhafte Materialien sind Glas, Acryl, Silikon, Hydrogel und PMMA. Alternativ kann die Linse 1 aus einem anderen Material sein.

Die bisher beschriebenen Ausführungsformen haben Hauptzonen $Z_i$ gleicher Fläche, also sogenannte Fresnel'sche Ringzonen, wobei für den äußeren Radius $r_i$ der i-ten Hauptzone $Z_i$ gilt:

$$r_i = r_1 \cdot i^{0,5} . \qquad\qquad (5)$$

Bei einer solchen Ausgestaltung der Linse 1 bleiben die einzelnen Hauptbrechkräfte $D_F$, $D_M$, $D_N$ im Wesentlichen unabhängig von der optischen Pupillengröße, sei diese z.B. durch eine Lochblende oder durch eine Augenpupille gegeben. Es kann allerdings auch gewünscht sein, dass die einzelnen Hauptbrechkräfte $D_F$, $D_M$, $D_N$ eine Abhängigkeit von der Pupillengröße aufweisen, was z.B. durch Wahl der Radien $r_i$ gemäß

$$r_i = r_1 \cdot i^{z} \quad \text{mit } z \neq 0,5 \qquad\qquad (6)$$

erreicht werden kann. So kann z.B. beabsichtigt sein, dass die Nahbrechkraft $D_N$ bei großer Pupille etwas größer ist als bei kleiner Pupille. Hiefür können die Hauptzonen $Z_i$ mit zunehmendem Abstand von der optischen Achse A kleiner werdende Flächen aufweisen ($z < 0,5$). Soll umgekehrt z.B. die Nahbrechkraft $D_N$ mit zunehmender Pupillengröße abnehmen, können die Hauptzonen $Z_i$ mit zunehmendem Abstand von der optischen Achse A größer werdende Flächen aufweisen ($z > 0,5$).

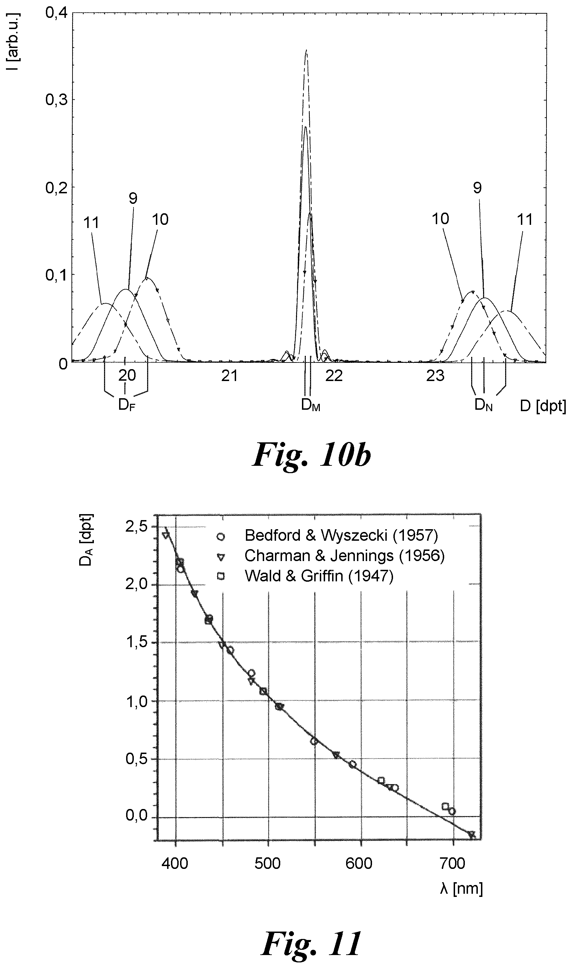

In den Fig. 10a und 10b sind Brechkraftverteilungen für verschiedene Werte des Parameters z bei monochromatischem Licht mit einer Wellenlänge von 550 nm (Fig. 10a) und bei polychromatischem Licht mit Wellenlängen von 450 nm bis 650 nm (Fig. 10b) dargestellt, für einen Wert z von 0,5 mit durchgezogenen Linien 10, für einen Wert z von 0,48 mit strichlierten Linien 11 und für einen Wert z von 0,52 mit Linien 12 mit Dreiecken. Wie ersichtlich ändern sich mit dem Parameter z die Hauptbrechkräfte $D_F$, $D_M$, $D_N$, deren zugehörige Intensitäten $I_F$, $I_M$, $I_N$ und ihre jeweiligen Maxima; die Summe der Intensitäten $I_F$, $I_M$, $I_N$ jedoch nicht.

Herkömmlicherweise gilt es als Vorteil, bei diffraktiven bi- oder trifokalen Linsen die diffraktive Brechkraft der nullten Diffraktionsordnung als Fernbrechkraft $D_F$ zu verwenden, da in der nullten Diffraktionsordnung keine diffraktive chromatische Aberration vorhanden ist. In einer Ausführungsform der hier offenbarten Linse 1 wird jedoch die diffraktive Brechkraft der negativen ersten Diffraktionsordnung nun als Fernbrechkraft $D_F$ verwendet, z.B. um die chromatische Aberration der Augenlinse nachzubilden, wie im Folgenden beschrieben.

In Fig. 11 ist die Brechkraft $D_A$ des menschlichen Auges in Abhängigkeit von der Wellenlänge $\lambda$ des Lichts dargestellt (nach: Charman WN, Jennings JAM (1976), "Objective Measurement of the longitudinal chromatic aberration of the human eye", Vision Res. 16:999 - 1005). Wie Fig. 11 zu entnehmen ist, beträgt die longitudinale chromatische Aberration des menschlichen Auges zwischen 450 nm und 650 nm ca. 1,3 Dioptrien, wobei die Brechkraft für 450 nm (blaues Licht) größer ist als für 650 nm (rotes Licht). Gemäß dem Standardwerk Bergmann - Schäfer: Optik, Verlag Walter de Gruyter, 1993 beträgt die Brechkraft des gesamten menschlichen Auges 58,8 Dioptrien und jene der Augenlinse 20,2 Dioptrien. Unter der Voraussetzung, dass die chromatischen Aberrationen des gesamten Auges und der darin befindlichen Augenlinse proportional zu den jeweiligen Brechkräften sind, kann die chromatische Aberration der Augenlinse größenordnungsmäßig als 1,3 · 20,2 / 58,8 = 0,447 ≈ 0,45 Dioptrien abgeschätzt werden, wobei blaues Licht stärker gebrochen wird als rotes.

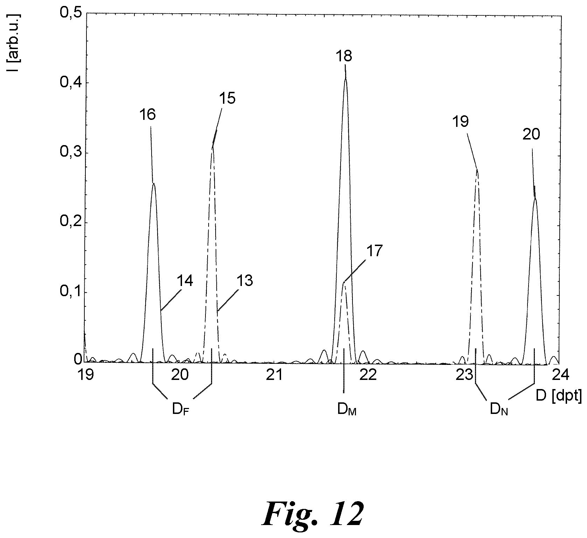

In Fig. 12 sind die Hauptbrechkräfte $D_F$, $D_M$, $D_N$ der Linse 1 der Fig. 2 und 3 für die beiden Wellenlängen 450 nm (strichlierte Kurve 13) und 650 nm (durchgezogene Kurve 14) dargestellt. Wie Fig. 12 zu entnehmen ist, ist für die beispielhaft gewählten Parameter die diffraktive longitudinale chromatische Aberration in der negativen ersten Diffraktionsordnung (für die Fernbrechkraft $D_F$) 0,51 Dioptrien (siehe die beiden linken Peaks 15, 16 in Fig. 12), ein Wert, der in der Nähe des oben für die Augenlinse angeführten Werts liegt. In der nullten Diffraktionsordnung (für die Mittelbrechkraft $D_M$) ist die diffraktive longitudinale chromatische Aberration der Linse 1 gleich Null (siehe die beiden mittleren Peaks 17, 18 in Fig. 12), und in der positiven ersten Diffraktionsordnung (für die Nahbrechkraft $D_N$) ist die diffraktive longitudinale chromatische Aberration -0,51 Dioptrien (siehe die beiden rechten Peaks 19, 20 in Fig. 12), d.h., dass rotes Licht stärker gebrochen wird als blaues, was der chromatischen Aberration des restlichen Auges entgegenwirkt. Damit weist die Linse 1 für die Ferne etwa die chromatische Aberration der Augenlinse auf, für die Mitteldistanz keine chromatische Aberration und für die Nähe etwa die chromatische Aberration der Augenlinse mit umgekehrtem Vorzeichen.

Die Parameter der Linse 1 können gemäß anderen Abschätzungen der augeneigenen chromatischen Aberration gewählt bzw. an die individuelle (vorher vermessene) Augenlinse eines Patienten angepasst werden. Z.B. könnte die negative erste Diffraktionsordnung der Linse für Licht zwischen 450 nm und 650 nm eine diffraktive longitudinale Aberration aufweisen, welche im Bereich von 0,1 bis 1,2 Dioptrien liegt, z.B. im Bereich von 0,3 bis 0,7 Dioptrien, insbesondere - nahe dem oben abgeschätzten Wert - im Bereich von 0,35 bis 0,55 Dioptrien.

Selbstverständlich können die refraktiven Brechkräfte $D_1$, $D_2$ der Subzonen 2, 3, deren flächenmäßige Anteile $p_1$, $p_2$ an ihrer jeweiligen Hauptzone $Z_i$ und die damit einhergehenden Hauptbrechkräfte $D_F$, $D_M$, $D_N$, ... der Linse 1 von den dargestellten Ausführungsformen abweichen bzw. mehrere der vorgestellten Ausführungsformen kombiniert werden. Beispielsweise kann die Differenz zwischen der Nahbrechkraft $D_N$ und der Fernbrechkraft $D_F$ im Bereich von 1 bis 6 Dioptrien liegen, z.B. im Bereich von 2,0 bis 4,5 Dioptrien. Dabei kann z.B. die Differenz zwischen der Mittelbrechkraft $D_M$ und der Fernbrechkraft $D_F$ im Bereich von 1,4 bis 2 Dioptrien liegen, etwa im Bereich von 1,6 bis 1,8 Dioptrien. Ferner kann eine der refraktiven Brechkräfte $D_1$ bzw. $D_2$ der Subzonen 2, 3 z.B. im Bereich von -2,5 bis 2,5 Dioptrien um die Fernbrechkraft $D_F$ liegen, insbesondere im Bereich von - 2,0 bis 2,0 Dioptrien; alternativ oder zusätzlich dazu könnte die andere der refraktiven Brechkräfte $D_1$ bzw. $D_2$ der Subzonen 2, 3 z.B. im Bereich von -2,5 bis 2,5 Dioptrien um die Nahbrechkraft $D_N$ liegen, insbesondere im Bereich von -2,0 bis 2,0 Dioptrien.

Die Erfindung ist nicht auf die dargestellten Ausführungsformen beschränkt, sondern umfasst alle Varianten, Modifikationen und Kombinationen, die in den Rahmen der angeschlossenen Ansprüche fallen.

**Patentansprüche**

1.   Multifokale Linse mit zumindest drei Hauptbrechkräften ($D_F$, $D_M$, $D_N$), welche mehrere konzentrische, jeweils aneinander angrenzende ringförmige Hauptzonen ($Z_i$) aufweist, von denen jede in eine innere und eine äußere ringförmige Subzone (2, 3) verschiedener refraktiver Brechkraft ($D_1$, $D_2$) unterteilt ist, und wobei die Linse (1) frei von geometrischen Stufen zwischen allen Subzonen (2, 3) ist,
**dadurch gekennzeichnet, dass** die refraktiven Brechkräfte ($D_1$) aller inneren Subzonen (2) untereinander jeweils gleich sind und die refraktiven Brechkräfte ($D_2$) aller äußeren Subzonen (3) untereinander jeweils gleich sind, und dass alle inneren und äußeren Subzonen (2, 3) ihre jeweilige Hauptzone ($Z_i$) in einem gleichen Flächenverhältnis teilen, welches im Bereich von 30:70 bis 70:30 liegt, wobei zumindest eine der Hauptbrechkräfte ($D_F$, $D_M$, $D_N$) diffraktiv ist.

2.   Multifokale Linse nach Anspruch 1, **dadurch gekennzeichnet, dass** alle Hauptzonen ($Z_i$) jeweils die gleiche Fläche haben.

3.   Multifokale Linse nach Anspruch 1, **dadurch gekennzeichnet, dass** die Flächen der Hauptzonen ($Z_i$) von innen nach

außen monoton zu- oder abnehmen.

4. Multifokale Linse nach einem der Ansprüche 1 bis 3, **dadurch gekennzeichnet, dass** die Fläche jeder Hauptzone ($Z_i$) kleiner als $2,2\pi$ mm$^2$ ist, bevorzugt kleiner als $\pi$ mm$^2$, besonders bevorzugt kleiner als $2\pi/3$ mm$^2$.

5. Multifokale Linse nach einem der Ansprüche 1 bis 4, **dadurch gekennzeichnet, dass** das genannte Flächenverhältnis im Bereich von 40:60 bis 60:40 liegt, bevorzugt 50:50 ist.

6. Multifokale Linse nach einem der Ansprüche 1 bis 5, **dadurch gekennzeichnet, dass** die Linse (1) 5 bis 50 Hauptzonen ($Z_i$) aufweist, bevorzugt 10 bis 20 Hauptzonen ($Z_i$).

7. Multifokale Linse nach einem der Ansprüche 1 bis 6, **dadurch gekennzeichnet, dass** die Linse (1) als Hauptbrechkräfte eine Fern-, eine Mittel- und eine Nahbrechkraft ($D_F$, $D_M$, $D_N$) aufweist, wobei die Differenz zwischen der Nahbrechkraft ($D_N$) und der Fernbrechkraft ($D_F$) im Bereich von 1 bis 6 Dioptrien liegt, bevorzugt im Bereich von 2,0 bis 4,5 Dioptrien.

8. Multifokale Linse nach Anspruch 7, **dadurch gekennzeichnet, dass** die Differenz zwischen der Mittelbrechkraft ($D_M$) und der Fernbrechkraft ($D_F$) im Bereich von 1,4 bis 2 Dioptrien liegt, bevorzugt im Bereich von 1,6 bis 1,8 Dioptrien.

9. Multifokale Linse nach einem der Ansprüche 1 bis 8, **dadurch gekennzeichnet, dass** alle Subzonen (2, 3) in Kombination ein Beugungsgitter bilden, das die zumindest drei Hauptbrechkräfte ($D_F$, $D_M$, $D_N$) der Linse (1) durch Diffraktion erzeugt, bevorzugt genau drei diffraktive Hauptbrechkräfte ($D_F$, $D_M$, $D_N$).

10. Multifokale Linse nach den Ansprüchen 7 und 9 oder nach den Ansprüchen 8 und 9, **dadurch gekennzeichnet, dass** die Fernbrechkraft ($D_F$) durch die negative erste Diffraktionsordnung der Linse (1) erzeugt ist.

11. Multifokale Linse nach Anspruch 10, **dadurch gekennzeichnet, dass** die negative erste Diffraktionsordnung der Linse (1) für Licht zwischen 450 nm und 650 nm eine diffraktive longitudinale chromatische Aberration aufweist, welche im Bereich von 0,1 bis 1,2 Dioptrien liegt, bevorzugt im Bereich von 0,3 bis 0,7 Dioptrien, besonders bevorzugt im Bereich von 0,35 bis 0,55 Dioptrien.

12. Multifokale Linse nach einem der Ansprüche 1 bis 11, **dadurch gekennzeichnet, dass** die Brechkraft ($D_1$) der inneren Subzonen (2) oder die Brechkraft ($D_2$) der äußeren Subzonen (3) im Bereich von -2,5 bis 2,5 Dioptrien um die kleinste der Hauptbrechkräfte ($D_F$, $D_M$, $D_N$) liegt, bevorzugt im Bereich von - 2,0 bis 2,0 Dioptrien um die kleinste der Hauptbrechkräfte ($D_F$, $D_M$, $D_N$).

13. Multifokale Linse nach einem der Ansprüche 1 bis 12, **dadurch gekennzeichnet, dass** die Brechkraft ($D_1$) der inneren Subzonen (2) oder die Brechkraft ($D_2$) der äußeren Subzonen (3) im Bereich von -2,5 bis 2,5 Dioptrien um die größte der Hauptbrechkräfte ($D_F$, $D_M$, $D_N$) liegt, bevorzugt im Bereich von -2, 0 bis 2,0 Dioptrien um die größte der Hauptbrechkräfte ($D_F$, $D_M$, $D_N$).

14. Multifokale Linse nach einem der Ansprüche 1 bis 13, **dadurch gekennzeichnet, dass** zumindest eine innere oder äußere Subzone (2, 3) in Teilzonen unterteilt ist, deren gemittelte refraktive Brechkräfte der refraktiven Brechkraft ($D_1$, $D_2$) der jeweiligen Subzone (2, 3) entsprechen.

## Claims

1. A multifocal lens having at least three main refractive powers ($D_F$, $D_M$, $D_N$), comprising a plurality of concentric annular main zones ($Z_i$) respectively adjacent to each other, each of which is divided into an inner and an outer annular subzone (2, 3) of different refractive power ($D_1$, $D_2$), and wherein the lens (1) is free of geometrical steps between all subzones (2, 3),
**characterised in that** the refractive powers ($D_1$) of all inner subzones (2) are in each case equal to one another and the refractive powers ($D_2$) of all outer subzones (3) are in each case equal to one another, and **in that** all inner and outer subzones (2, 3) share their respective main zone ($Z_i$) in an equal area ratio, which is in the range from 30:70 to 70:30, wherein at least one of the main refractive powers ($D_F$, $D_M$, $D_N$) is diffractive.

2. The multifocal lens according to claim 1, **characterised in that** all the main zones ($Z_i$) each have the same area.

3. The multifocal lens according to claim 1, **characterised in that** the areas of the main zones ($Z_i$) increase or decrease monotonically from the inside to the outside.

4. The multifocal lens according to any one of claims 1 to 3, **characterised in that** the area of each main zone ($Z_i$) is less than $2.2\pi$ mm$^2$, preferably less than $\pi$ mm$^2$, particularly preferably less than $2\pi/3$ mm$^2$.

5. The multifocal lens according to any one of claims 1 to 4, **characterised in that** said area ratio is in the range of 40:60 to 60:40, preferably 50:50.

6. The multifocal lens according to any one of claims 1 to 5, **characterised in that** the lens (1) has 5 to 50 main zones ($Z_i$), preferably 10 to 20 main zones ($Z_i$).

7. The multifocal lens according to any one of claims 1 to 6, **characterised in that** the lens (1) has as main refractive powers a far, a middle and a near refractive power ($D_F$, $D_M$, $D_N$), wherein the difference between the near refractive power ($D_N$) and the far refractive power ($D_F$) is in the range from 1 to 6 dioptres, preferably in the range from 2.0 to 4.5 dioptres.

8. The multifocal lens according to claim 7, **characterised in that** the difference between the middle refractive power ($D_M$) and the far refractive power ($D_F$) is in the range from 1.4 to 2 dioptres, preferably in the range from 1.6 to 1.8 dioptres.

9. The multifocal lens according to any one of claims 1 to 8, **characterised in that** all subzones (2, 3) in combination form a diffraction grating which generates the at least three main refractive powers ($D_F$, $D_M$, $D_N$) of the lens (1) by diffraction, preferably exactly three diffractive main refractive powers ($D_F$, $D_M$, $D_N$).

10. The multifocal lens according to claims 7 and 9 or according to claims 8 and 9, **characterised in that** the far refractive power ($D_F$) is generated by the negative first diffraction order of the lens (1).

11. The multifocal lens according to claim 10, **characterised in that** the negative first diffraction order of the lens (1) for light between 450 nm and 650 nm has a diffractive longitudinal chromatic aberration which is in the range from 0.1 to 1.2 dioptres, preferably in the range from 0.3 to 0.7 dioptres, particularly preferably in the range from 0.35 to 0.55 dioptres.

12. The multifocal lens according to any one of claims 1 to 11, **characterised in that** the refractive power ($D_1$) of the inner subzones (2) or the refractive power ($D_2$) of the outer subzones (3) is in the range from -2,5 to 2.5 dioptres around the smallest of the main refractive powers ($D_F$, $D_M$, $D_N$), preferably in the range from -2.0 to 2.0 dioptres around the smallest of the main refractive powers ($D_F$, $D_M$, $D_N$).

13. The multifocal lens according to any one of claims 1 to 12, **characterised in that** the refractive power ($D_1$) of the inner subzones (2) or the refractive power ($D_2$) of the outer subzones (3) is in the range from -2,5 to 2.5 dioptres around the largest of the main refractive powers ($D_F$, $D_M$, $D_N$), preferably in the range from -2.0 to 2.0 dioptres around the largest of the main refractive powers ($D_F$, $D_M$, $D_N$).

14. The multifocal lens according to any one of claims 1 to 13, **characterised in that** at least one inner or outer subzone (2, 3) is subdivided into partial zones of which the averaged refractive powers correspond to the refractive power ($D_1$, $D_2$) of the respective subzone (2, 3).

**Revendications**

1. Lentille multifocale avec au moins trois pouvoirs réfringents principaux ($D_F$, $D_M$, $D_N$), laquelle présente plusieurs zones principales ($Z_i$) concentriques de forme annulaire respectivement adjacentes les unes aux autres, parmi lesquelles chacune est subdivisée en sous-zones (2, 3) de forme annulaire intérieure et extérieure de pouvoir réfringent ($D_1$, $D_2$) différent, et où la lentille (1) est exempte de niveaux géométriques entre toutes les sous-zones (2, 3),
   **caractérisée en ce que** les pouvoirs réfringents ($D_1$) de toutes les sous-zones intérieures (2) sont à chaque fois respectivement égaux les uns aux autres et les pouvoirs réfringents ($D_2$) de toutes les sous-zones extérieures (3) sont à chaque fois respectivement égaux les uns aux autres et que toutes les sous-zones (2, 3) intérieures et extérieures partagent leur zone principale ($Z_i$) respective dans un même rapport de surface, lequel se situe dans la plage de 30 : 70 à 70 : 30, où au moins un des pouvoirs réfringents principaux ($D_F$, $D_M$, $D_N$) est diffractif.

2. Lentille multifocale selon la revendication 1, **caractérisée en ce que** toutes les zones principales ($Z_i$) possèdent respectivement la même surface.

3. Lentille multifocale selon la revendication 1, **caractérisée en ce que** les surfaces des zones principales ($Z_i$) augmentent ou diminuent de manière monotone de l'intérieur vers l'extérieur.

4. Lentille multifocale selon l'une des revendications 1 à 3, **caractérisée en ce que** la surface de chaque zone principale ($Z_i$) est inférieure à $2,2\pi$ mm$^2$, de préférence inférieure à $\pi$ mm$^2$, de manière particulièrement préférée inférieure à $2\pi/3$ mm$^2$.

5. Lentille multifocale selon l'une des revendications 1 à 4, **caractérisée en ce que** ledit rapport de surface se situe dans la plage de 40 : 60 à 60 : 40, de préférence à 50 : 50.

6. Lentille multifocale selon l'une des revendications 1 à 5, **caractérisée en ce que** la lentille (1) présente de 5 à 50 zones principales ($Z_i$), de préférence de 10 à 20 zones principales ($Z_i$).

7. Lentille multifocale selon l'une des revendications 1 à 6, **caractérisée en ce que** la lentille (1) présente, en tant que pouvoirs réfringents principaux, un pouvoir réfringent de loin, un pouvoir réfringent moyen et un pouvoir réfringent de près ($D_F$, $D_M$, $D_N$) , dans laquelle la différence entre le pouvoir réfringent de près ($D_N$) et le pouvoir réfringent de loin ($D_F$) se situe dans la plage de 1 à 6 dioptries, de préférence dans la plage de 2,0 à 4,5 dioptries.

8. Lentille multifocale selon la revendication 7, **caractérisée en ce que** la différence entre le pouvoir réfringent moyen ($D_M$) et le pouvoir réfringent de loin ($D_F$) se situe dans la plage de 1,4 à 2 dioptries, de préférence dans la plage de 1,6 à 1,8 dioptries.

9. Lentille multifocale selon l'une des revendications 1 à 8, **caractérisée en ce que** toutes les sous-zones (2, 3) en combinaison forment un réseau de diffraction qui crée par diffraction les au moins trois pouvoirs réfringents principaux ($D_F$, $D_M$, $D_N$) de la lentille (1), de préférence exactement trois pouvoirs réfringents principaux ($D_F$, $D_M$, $D_N$) diffractifs.

10. Lentille multifocale selon les revendications 7 et 9 ou selon les revendications 8 et 9, **caractérisée en ce que** le pouvoir réfringent de loin ($D_F$) est créé par le premier ordre de diffraction négatif de la lentille (1).

11. Lentille multifocale selon la revendication 10, **caractérisée en ce que** le premier ordre de diffraction négatif de la lentille (1) présente, pour de la lumière entre 450 nm et 650 nm, une aberration chromatique longitudinale diffractive, laquelle se situe dans la plage de 0,1 à 1,2 dioptries, de préférence dans la plage de 0,3 à 0,7 dioptries, de manière particulièrement préférée dans la plage de 0,35 à 0,55 dioptries.

12. Lentille multifocale selon l'une des revendications 1 à 11, **caractérisée en ce que** le pouvoir réfringent ($D_1$) des sous-zones intérieures (2) ou le pouvoir réfringent ($D_2$) des sous-zones extérieures (3) se situe dans la plage de -2,5 à 2,5 dioptries autour du plus petit des pouvoirs réfringents principaux ($D_F$, $D_M$, $D_N$), de préférence dans la plage de -2,0 à 2,0 dioptries autour du plus petit des pouvoirs réfringents principaux ($D_F$, $D_M$, $D_N$).

13. Lentille multifocale selon l'une des revendications 1 à 12, **caractérisée en ce que** le pouvoir réfringent ($D_1$) des sous-zones intérieures (2) ou le pouvoir réfringent ($D_2$) des sous-zones extérieures (3) se situe dans la plage de -2,5 à 2,5 dioptries autour du plus grand des pouvoirs réfringents principaux ($D_F$, $D_M$, $D_N$), de préférence dans la plage de -2,0 à 2,0 dioptries autour du plus grand des pouvoirs réfringents principaux ($D_F$, $D_M$, $D_N$).

14. Lentille multifocale selon l'une des revendications 1 à 13, **caractérisée en ce qu'**au moins une sous-zone (2, 3) intérieure ou extérieure est subdivisée en zones partielles dont les pouvoirs réfringents moyennés correspondent au pouvoir réfringent ($D_1$, $D_2$) de la sous-zone (2, 3) respective.

**Fig. 1**
*(Stand der Technik)*

**Fig. 2**

**Fig. 3**

**Fig. 4**

*Fig. 5*

*Fig. 6*

**Fig. 7**

**Fig. 8**

*Fig. 9*

*Fig. 10a*

15

Fig. 10b

Fig. 11

*Fig. 12*

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102020215362 A1
- US 5760871 A
- EP 1194797 B1
- EP 2564265 B1

**In der Beschreibung aufgeführte Nicht-Patentliteratur**

- **CHARMAN WN** ; **JENNINGS JAM**. Objective Measurement of the longitudinal chromatic aberration of the human eye. *Vision Res*, 1976, vol. 16, 999-1005